(19) Europäisches Patentamt
European Patent Office
Office européen des brevets

(11) Publication number: **0 122 641**
**B1**

(12) # EUROPEAN PATENT SPECIFICATION

(45) Date of publication of patent specification: **14.03.90**

(51) Int. Cl.$^5$: **C 12 Q 1/28,** G 01 N 33/52

(21) Application number: **84104415.9**

(22) Date of filing: **18.04.84**

(54) **Analytical reagent, analytical method, and multilayer chemical-analytical element.**

(30) Priority: **18.04.83 JP 68009/83**

(43) Date of publication of application:
**24.10.84 Bulletin 84/43**

(45) Publication of the grant of the patent:
**14.03.90 Bulletin 90/11**

(84) Designated Contracting States:
**DE GB**

(56) References cited:
**US-A-4 063 894**

**CHEMICAL ABSTRACTS, vol. 99, o. 25, 19th December 1983, page 361, abstract no. 209284j, Columbus, Ohio, US; & JP-A-58 45 557 (YATORON K.K.) 16-03-1983**

**RESEARCH DISCLOSURE, no. 160, August 1977, pages 19-24, disclosure no. 16034, Homewell Havant, Hampshire, GB: "Compositions for the detection of hydrogen peroxide"**

(73) Proprietor: **FUJI PHOTO FILM CO., LTD.**
**210 Nakanuma Minami Ashigara-shi**
**Kanagawa 250-01 (JP)**

(72) Inventor: **Sato, Akira**
**c/o Fuji Photo Film Co., Ltd. 3-11-46, Senzui**
**Asaka-shi Saitama (JP)**
Inventor: **Osada, Chiaki**
**c/o Fuji Photo Film Co., Ltd. 3-11-46, Senzui**
**Asaka-shi Saitama (JP)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 122 641 B1

**Description**

BACKGROUND OF THE INVENTION

Field of the invention

The present invention relates to a novel reagent for analysis of hydrogen peroxide, a method of analysis of hydrogen peroxide using the same, a method of analysis of peroxidase using the same, and a multilayer chemical-analytical element containing the same. More particularly, the present invention relates to a novel color forming compound which is capable of forming a color in the presence of hydrogen peroxide and a catalytic substance having an oxidizing activity, such as peroxidase, a method for colorimetric quantitative analysis of hydrogen peroxide using the same, and a multilayer chemical-analytical element which contains the same and is suitably employable in quantitative analysis of hydrogen peroxide or an analyte (substance to be analyzed) capable of forming hydrogen peroxide, as well as in quantitative analysis of peroxidase activity.

Description of prior arts

Enzymic analytical methods in clinical test have been highly valued in their specific reactivity and have rapidly come into wide use in recent years. Particularly, in quantitative analyses of glucose, uric acid, cholesterol, triglyceride, lactic acid, creatinine, free fatty acid, glutamate-pyruvate transaminase, glutamateoxaloacetate transaminase, cholinesterase, creatine phosphokinase and lactate dehydrogenase in body fluid and urine, there are frequently used methods of quantitatively analyzing such object (analyte) by causing said analyte or a final intermediate product derived from the analyte to react with an oxidase therefor and determining thus produced hydrogen peroxide.

As the methods of quantitative determination of hydrogen peroxide, there are known, for example, methods disclosed in the specifications of Japanese Patent Provisional Publication Nos. 50(1975)—115892, 53(1975)—110897, 54(1979)—25892, 55(1980)—20471, 55(1980)—92696, 55(1980)—121149, 55(1980)—131400, 56(1981)—37557, 56(1981)—39072, 56(1981)—42599, 57(1982)—142562, 57(1982)—150399, etc. These specifications disclose colorimetric methods comprising converting a chromogen such as o-tolidine, 2,7-diaminofluorene, N,N-dimethyl-p-phenylenediamine, o-dianisidine or o-aminophenol into a colored substance through oxidation, as well as colorimetric methods comprising converting a chromogen such as a combination of 4-aminoantipyrine with phenol, N,N-dialkylaniline or N,N-dialkyltoluidine, a combination of 3-methyl-2-benzthiazolinone hydrazone with o-tolidine, N,N-dimethylaniline or N,N-diethylaniline, or 4-methoxy-1-naphthol capable of forming a dimer or its derivative into a colored substance by oxidative condensation.

The methods using these compounds are disadvantageous in determination of minor constituents (analyte), since these compounds require two molar hydrogen peroxide to form one molar colored substance (dye). For this reason, a reagent for quantitatively analyzing hydrogen peroxide, which requires only one molar hydrogen peroxide to form one molar dye is desired.

SUMMARY OF THE INVENTION

An object of the present invention is to provide a highly sensitive reagent advantageously employable for quantitative determination of hydrogen peroxide, which can form one molar dye with one molar hydrogen peroxide under the catalytic action or coupling reaction of peroxidase.

Another object of the present invention is to provide a method of quantitative determination of hydrogen peroxide using the above-mentioned reagent.

A further object of the present invention is to provide a multilayer chemical-analytical element for quantitative determination of hydrogen peroxide, which has a reagent layer containing the above-identified reagent.

The present inventors have discovered that the above objects can be achieved using a compound having the formula (I):

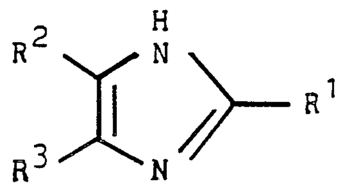

(I)

wherein $R^1$ is an aryl group substituted with hydroxyl group at the ortho- or para-position, $R^2$ is an unsubstituted or substituted aryl group, and $R^3$ is an unsubstituted or substituted alkyl or an unsubstituted alkenyl group.

DETAILED DESCRIPTION OF THE INVENTION

The hydroxyl group-substituted aryl group represented by the definition $R^1$ of the formula (I) may further have one or more substituents selected from the group consisting of halogen (chlorine, bromine,

etc.), cyano, alkoxy, monoalkylamino and dialkylamino groups, and when this aryl group has two or more substituents, they may be the same or different from each other. The substituted aryl groups represented by $R^2$ may have one or more substituents selected from the group consisting of halogen (chlorine, bromine, etc.), hydroxyl, cyano, alkoxy, monoalkylamino and dialkylamino groups, and when the aryl group has two or more substituents, they may be the same or different from each other.

The substituted alkyl group represented by $R^3$ may have one or more substituents selected from the group consisting of hydroxyl, alkoxy, aryloxy, cyano, monoalkylamino, dialkylamino, carboxy, alkoxycarbonyl, carbamoyl, N-alkylcarbamoyl, N,N-dialkylcarbamoyl, alkylsulfonyl, sulfamoyl, N-alkylsulfamoyl, N,N-dialkylsulfamoyl and aryl groups, and aryl and aryloxy groups are preferred. When the alkyl group has two or more substituents, they may be the same or different group from each other.

When $R^3$ is an alkyl group, there is no critical upper limit for the number of carbon atoms, but alkyl groups having at least two carbon atoms, particularly at least 6 carbon atoms are preferred. When the number of carbon atoms is three or more, the carbon chain may be in any of straight, branched and cyclic forms. When $R^3$ is an alkenyl group, it contains at least two carbon atoms and there is no critical upper limit for the number of carbon atoms, but alkenyl groups having at least 6 carbon atoms are preferred. When the number of carbon atoms is three or more, the carbon chain may be in any of straight and branched forms, and when the number is five or more, the carbon chain may be in a cyclic form.

Preferred examples of the substituted aryl group represented by $R^1$ are 2-hydroxyphenyl, 4-hydroxyphenyl, 3,5-dibromo-4-hydroxyphenyl, 3-bromo-4-hydroxy-5-methoxyphenyl, 3-methoxy-4-hydroxyphenyl and 3,5-dimethoxy-4-hydroxyphenyl groups.

Preferred examples of the substituents represented by $R^2$ are phenyl, 4-methoxyphenyl, 4-ethoxyphenyl, 4-(dimethylamino)phenyl, 4-(diethylamino)phenyl groups and those described above as the preferred example of $R^1$.

Preferred examples of the unsubstituted alkyl groups of $R^3$ are ethyl, propyl, butyl, hexyl, octyl, decyl, isopropyl, isoamyl, cyclohexyl, cyclohexylmethyl and cyclohexylethyl groups. Among them, octyl, decyl and cyclohexylethyl groups are particularly preferred.

Preferred examples of the substituted alkyl groups of $R^3$ are benzyl, phenethyl, 3-phenylpropyl, phenoxymethyl, 2-phenoxyethyl and 3-phenoxypropyl groups. Among them, phenethyl, 3-phenylpropyl, 2-phenoxyethyl and 3-phenoxypropyl groups are particularly preferred, because they are easily soluble in water and give compounds showing less interlayer diffusion when incorporated in the multilayer chemical-analytical element.

Preferred examples of the alkenyl groups of $R^3$ are 3-butenyl, 4-pentenyl and 5-hexenyl groups.

Preferred compounds of the formula (I) are illustrated below. However, it is not intended that the compounds of the present invention be limited to the illustrated compounds. In the following formulas, Me represents methyl, Et represents ethyl, Ph represents phenyl and Bz represents benzyl.

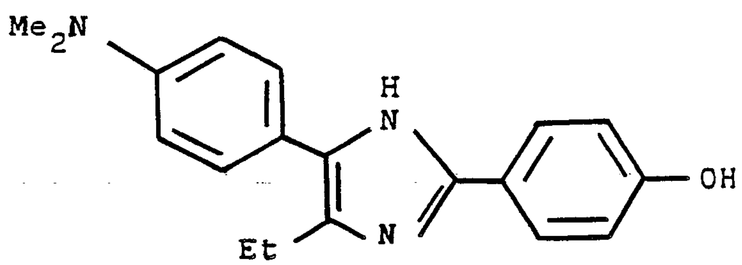

(1)

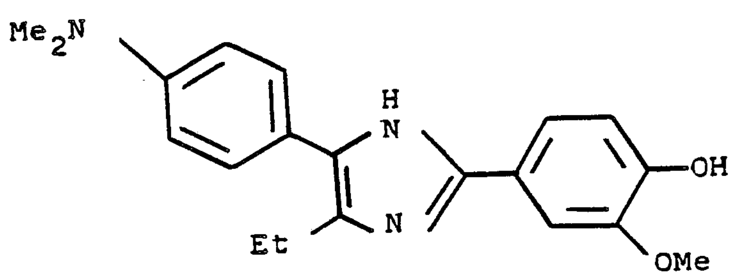

(2)

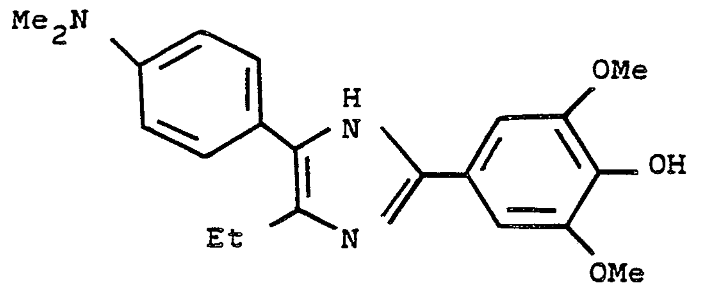

(3)

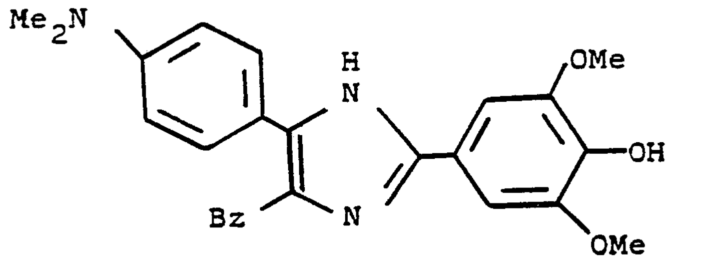

(4)

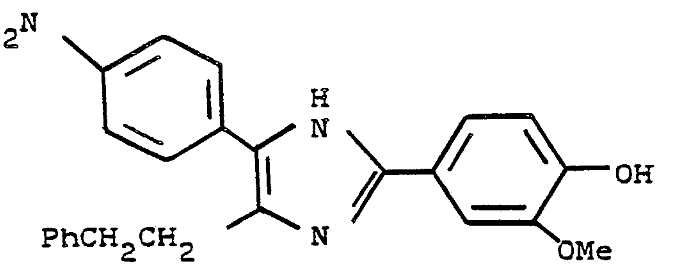

(5)

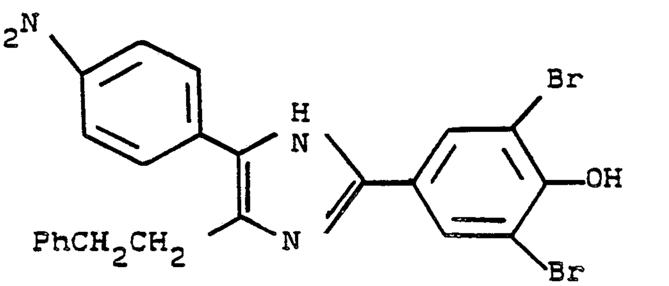

(6)

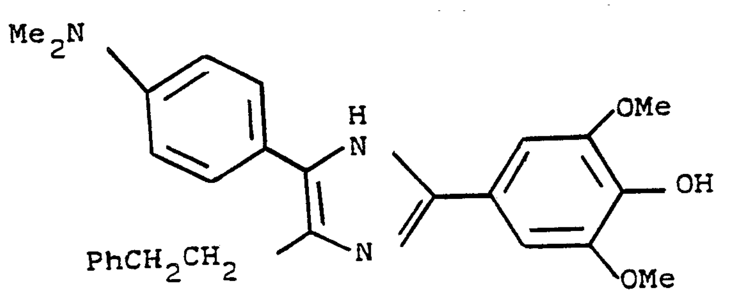

(7)

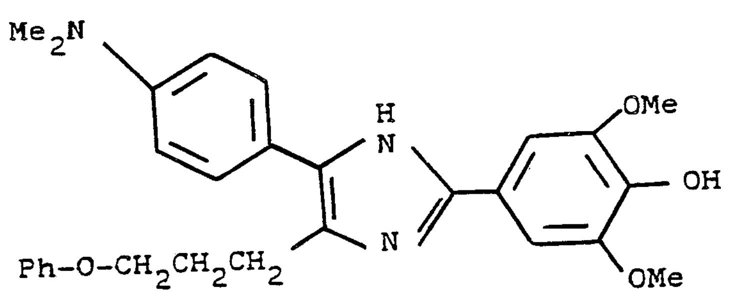

(8)

Among these compounds, the compound (7) is preferred, because it has high solubility in water and exhibits less interlayer diffusion when incorporated in a multilayer chemical-analytical element.

The novel compounds having the formula (I) can be synthesized according to the method described in U.S. Patent No. 3,297,710. The following synthesis examples illustrate the preparation of the compounds having the formula (I). The compounds which are not exemplified can be synthesized in similar manners to those of the synthesis examples.

Synthesis Example 1

Synthesis of 2-(3,5-dimethoxy-4-hydroxyphenyl)-4-[4-(dimethylamino)phenyl]-5-benzylimidazole (Compound 4).

2.6 g. of 1-[4-(dimethylamino)phenyl]-3-phenylpropane-1,2-dione, 2 g. of syringaldehyde (i.e., 4-hydroxy-3,5-dimethoxybenzaldehyde) and 9 g. of ammonium acetate were refluxed in 100 ml of glacial acetic acid for two hours by heating to effect the reaction. After cooling, 300 ml of water was added. While cooling with ice, the reaction mixture was neutralized with aqueous ammonia to precipitate crystals. The crystals were collected by filtration, washed with water, dried and treated with benzene to afford 0.8 g. of the compound (4) as white crystals. Melting point: higher than 180°C (dec.).

Synthesis Example 2

Synthesis of 2-(3,5-dimethoxy-4-hydroxyphenyl)-4-[4-(dimethylamino)phenyl]-5-phenethyl-imidazole (Compound 7).

3 g. of 1-[4-(dimethylamino)phenyl]-4-phenylbutane-1,2-dione, 2 g. of syringaldehyde and 12 g. of ammonium acetate were refluxed in 100 ml of glacial acetic acid for two hours by heating to effect the reaction. Then the procedure of Synthesis Example 1 was repeated to afford 0.6 g. of the compound (7). Melting point: higher than 125°C (dec.).

The reagent for analysis of hydrogen peroxide in the present invention is prepared in such a manner that each component is prepared in the form of independent solid material (powder or granule), an aqueous solution or an aqueous buffer solution, and when used, that is, when a sample to be analyzed is subjected to quantitative analysis, these components are combined together and used. Alternatively, a mixture of all solid materials (in the form of powder or granule), or an aqueous solution or an aqueous buffer solution containing all components can be previously prepared and used. When the reagent is used in the form of a solution containing all components, the aqueous solution or the aqueous buffer solution can be prepared by dissolving each component simultaneously or in an appropriate order.

The reagent for analysis of hydrogen peroxide in the present invention is particularly suitable for use in an integral multilayer chemical-analytical element (film or sheet) wherein a reagent layer and a porous spreading layer (or a porous layer having a definite surface area) in this order are provided on a water-impermeable, light-transmissive support. In a preferred embodiment, each component of the reagent of the present invention is contained in the reagent layer (or said reagent layer and other layer which is provided, if necessary) of said element. Such application is disclosed in Japanese Patent Provisional Publication Nos. 49(1974)—53888 [USP 3,992,158], 50(1975)—137192 [USP 3,983,005], 51(1976)—40191 [USP 4,042,335], 55(1980)—90859, 55(1980)—164356 [USP 4,292,272], and 57(1982)—66359, Japanese Utility Model Provisional Publication No. 57(1982)—42951, etc.

The reaction (color-forming reaction) between the reagent of the present invention and hydrogen peroxide can be illustrated, for example, by the equation (II).

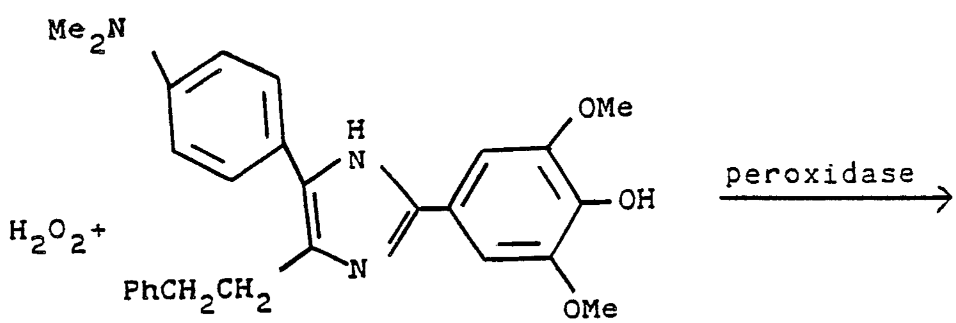

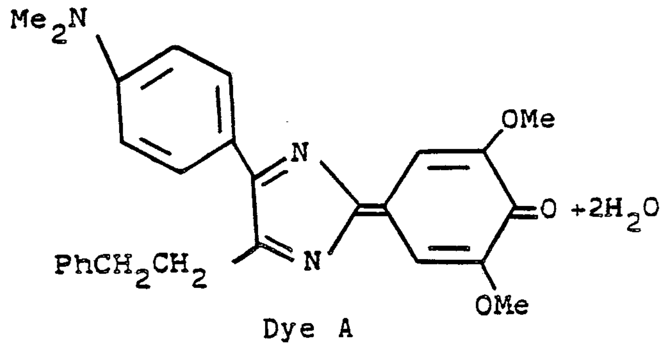

Dye A

Since a dye (dye A) is stoichiometrically formed by the reaction according to the equation (II), hydrogen peroxide can be quantitatively determined using a previously prepared calibration curve after measurement of the formed color through reflection or absorbance photometry.

The reagent of the present invention is characterized in that hydrogen peroxide produced from an analyte can be quantitatively determined with high sensitivity, because the reagent can produce one molar dye from one molar hydrogen peroxide.

The analytical method of the present invention can be carried out in the following manner. An imidazole derivative having the formula (I) and peroxidase (reaction catalyst) in the form as mentioned above are added simultaneously or in appropriate order to a sample to be analyzed. The reaction of the mixture is allowed to proceed at a temperature of from 5°C to 40°C, preferably 20°C to 40°C at a pH of from 3.0 to 9.0, preferably 4.5 to 8.0 for one to 60 minutes, and the absorbance of the reaction mixture containing the formed color is photometrically measured. Alternatively, the imidazole derivative and peroxidase are incorporated in the multilayer chemical-analytical element (film or sheet) described in the aforementioned patent specifications. About 6 to about 15 µl. of a sample solution to be analyzed is spotted on the porous spreading layer of said element, or a certain amount of the sample solution in the range of about 10 to about 30 µl. is spotted on the porous layer having a definite surface area, and incubated at a temperature of from 20°C to 40°C, preferably 25°C to 40°C for one to twenty minutes, preferably two to ten minutes, and the optical density of the colored area of the multilayer chemical-analytical element is measured through reflection photometry. The measurement of absorbance or reflection optical density can be conducted by any of an end point method and a reaction rate method.

The analytical reagent, the analytical method and the multilayer chemical-analytical element of the present invention are particularly suitable for use in the determination of hydrogen peroxide in a sample containing a trace amount of hydrogen peroxide (or an analyte capable of forming hydrogen peroxide) as an analyte.

By using an oxidase in combination with the imidazole derivative of the formula (I) and peroxidase in the present invention, said analytical reagent can be employed more widely. When an analyte or a final intermediate product derived from the analyte can produce hydrogen peroxide under the action of an oxidase, the oxidase is incorporated in the reagent of the present invention so that it becomes possible to quantitatively determine various substances through quantitative determination of the produced hydrogen peroxide. Practically, glucose, uric acid, cholesterol, triglyceride, lactic acid, creatinine, free fatty acid, glutamate-pyruvate transaminase, glutamate-oxaloacetate transaminase, cholinesterase, creatin phosphokinase and lactate dehydrogenase in body fluid (such as blood) and urine, etc. can be determined. Examples of said oxidases which can be used for the measurement include glucose oxidase, uricase, cholesterol oxidase, L-α-glyxerophosphate oxidase and pyruvate oxidase. The oxidases may be derived from any origin.

When an oxidase is used in combination, $H_2O_2$ may be previously produced under the action of the oxidase, but it is preferred that the $H_2O_2$-producing reaction and color reaction are allowed to proceed simultaneously. The amount of the oxidase to be used varies depending on the type of the oxidase, the substrate to be analyzed and other contained materials.

The peroxidase used in the present invention serves as a catalyst for an oxidation reaction of hydrogen donor by hydrogen peroxide.

Examples of peroxidases suitable for use in the present invention are peroxidase (EC 1.11.1.7) derived from vegetable and animal disclosed in Japanese Patent Publication Nos. 56(1981)—45599 [USP 3,983,005], 56(1982)—5520, etc. and peroxidase (EC 1.11.1.7) derived from microorganism disclosed in Japanese Patent Provisional Publication No. 57(1982)—99192, etc. Such peroxidase may be used either alone or as a mixture of two or more of them.

Examples of peroxidases derived from vegetable include those extracted from horse radish, potato, fig sap juice, turnip and radish. Examples of peroxidases derived from animal include lactoperoxidase extracted from milk and verdoperoxidase extracted from leucocyte. Examples of peroxidases derived from microorganism include those derived from microorganism possessing nonspecific peroxidase production ability and belonging to genera Alternaria, Cochliobolus, Curvularia and Pellicularia.

Further, there can be used inorganic compounds having peroxidase activity, such as iron thiocyanate, iron tannate, iron ferrocyanide (all compounds being divalent (+2) iron compounds), potassium chromate sulfate, sodium iodide, potassium iodide, ammonium molybdate and potassium molybdate disclosed in Japanese Patent Publication Nos. 56(1981)—45599, 57(1982)—5520, etc.

Among them, nonspecific peroxidases derived from vegetable or microorganism are preferred.

As shown in the reaction equation (II), an imidazole compound having the formula (I) reacts with hydrogen peroxide in a stoichiometric amount under the catalytic action of peroxidase to form a dye so that it is possible to analyze or quantitatively determine the peroxidase activity in a sample by adding or spotting the sample having unknown peroxidase activity to or on a reagent comprised of the imidazole compound having the formula (I) and $H_2O_2$ in combination or a multilayer chemical-analytical element containing both. This method can be applied to enzyme-immunoassay using peroxidase as a label substance.

It should be understood that the present invention can be applied to analyze hydrogen peroxide or an analyte capable of producing hydrogen peroxide in body fluid such as blood and urine of not only human being, but also animals such as poultry, pet and laboratory animals.

The following examples are given to illustrate the present invention in more detail.

Example 1

(1) Preparation of color-forming solution

A color-forming solution was prepared so as to make the total volume 25 ml by dissolving 15 mg. of the compound (3) in acetone and dissolving 5,000 U of peroxidase in an aqueous borate buffer solution (pH 7.5).

(2) Determination of hydrogen peroxide

3 ml of the color-forming solution was placed in a sample cell and 20 µl. of hydrogen peroxide having a known content was added thereto. As control, a blank solution was prepared by adding distilled water in place of the aqueous hydrogen peroxide solution. After standing at 37°C for 10 seconds, the absorbance was photometrically measured at a wavelength of 640 nm. The results are set forth in the following table.

| Content of Hydrogen Peroxide (M/l) | Absorbance |
|---|---|
| 0 (blank) | 0.12 |
| $8 \times 10^{-4}$ | 0.19 |
| $1 \times 10^{-3}$ | 0.25 |
| $2 \times 10^{-3}$ | 0.53 |
| $4 \times 10^{-3}$ | 1.06 |

Example 2

The surface of a transparent polyethylene terephthalate (PET) film (thickness 185 µm) having a gelatin subbing layer was coated with a coating solution for formation of a reagent layer consisting of the following components, and dried.

Numerical values given below are expressed by the content of each component in each layer after drying.

Components of Coating Solution for Reagent Layer
(solvent: appropriate amount of water)

| | |
|---|---|
| Uricase | 150 U/$m^2$ |
| Compound (7) | 0.2 g/$m^2$ |
| Peroxidase | 2,500 U/$m^2$ |
| Gelatin | 10 g/$m^2$ |
| pH 8.5 Borate buffer solution | |

The prepared reagent layer was wetted with water. A cellulose acetate membrane filter (average pore size 3.0 µm, thickness 180 µm, Microfilter FM—300 (trademark) manufactured by Fuji Photo Film Co., Ltd., Japan) serving as a porous spreading layer was laminated thereon to obtain an integrated multilayer chemical-analytical element for quantitative determination of uric acid.

For evaluating the resulting multilayer chemical-analytical element, 10 µl. of each of aqueous uric acid solutions (content; 6, 10 and 12 mg/dl) and a blank solution containing no uric acid, was spotted on the porous spreading layer and incubated at 37°C for two minutes. Immediately thereafter, the optical density of the formed color was measured at a measuring wavelength in the vicinity of 630 nm from the PET film side through reflection photometry by means of Macbeth reflection densitometer equipped with a cyan filter. Thus, the following results were obtained.

| Uric Acid Solution (content. mg/dl) | Reflection Optical Density |
|---|---|
| 0 (blank) | 0.43 |
| 6 | 0.79 |
| 10 | 0.95 |
| 12 | 1.05 |

It is apparent from the results that the content of uric acid can be determined by reflection photometry and colorimetry using the above multilayer chemical-analytical element.

Example 3

The surface of a transparent PET film (thickness 185 µm) having a gelatin subbing layer was coated with a coating solution for formation of a reagent layer consisting of the following components, and dried.

Components of Coating Solution of Reagent Layer
(solvent: appropriate amount of water)

| | |
|---|---|
| Uricase | 200 U/$m^2$ |
| Compound (7) | 0.25 g/$m^2$ |
| Gelatin | 10 g/$m^2$ |
| Peroxidase | 3000 U/$m^2$ |
| Octylphenoxypolyethoxyethanol [Triton X—100 (trade mark)] | 0.15 g/$m^2$ |
| pH 8.5 Borate buffer solution | |

The surface of the resulting reagent layer was coated with a coating dispersion for formation of a light-reflecting layer consisting of the following components, and dried.

Components of Coating Solution for Light-Reflecting
Layer (dispersing medium: appropriate amount of water)

| | |
|---|---|
| Particulate titanium dioxide powder | 3 g/$m^2$ |
| Gelatin | 1 g/$m^2$ |
| Octylphenoxypolyethoxyethanol | 0.1 g/$m^2$ |

Further, the surface of the resulting light-reflecting layer was coated with a coating solution for formation of an interfering substance-removing layer consisting of the following components, and dried.

Components of Coating Solution for Interfering Substance-Removing Layer (solvent: appropriate amount of water)

| | |
|---|---|
| Ascorbate oxidase | 4,000 $U/m^2$ |
| Gelatin | 4 $g/m^2$ |
| pH 8.5 Borate buffer solution | |

The resulting interfering substance-removing layer was wetted with water. A No. 100S count cotton broadcloth (a cloth which was washed with water, defatted and dried) was laminated thereon to obtain an integrated multilayer chemical-analytical element for quantitative determination of uric acid.

In a similar manner to that described in Example 2, the reflection optical density of a color formed from uric acid of known content was measured on the multilayer chemical-analytical element.

| Uric Acid Solution (content. mg/dl) | Reflection Optical Density |
|---|---|
| 0 (blank) | 0.25 |
| 6 | 0.50 |
| 10 | 0.64 |
| 12 | 0.73 |

Example 4

The surface of a transparent PET film (thickness 185 µm) having a gelatin subbing layer was coated with a coating solution for formation of a reagent layer consisting of the following components, and dried to form a second reagent layer.

Components of Coating Solution for Second Reagent Layer (solvent: appropriate amount of water)

| | |
|---|---|
| Peroxidase | 5,000 $U/m^2$ |
| Compound (7) | 1 $g/m^2$ |
| Gelatin | 10 $g/m^2$ |
| Octylphenoxypolyethoxyethanol [Triton X—100 (trade mark)] | 0.15 $g/m^2$ |
| pH 6.5 Borate buffer solution | |

The surface of the resulting reagent layer was coated with a coating dispersion for formation of a light-reflecting layer consisting of the following components, and dried to form a light-reflecting layer.

Components of Coating Dispersion for Light-Reflecting Layer (dispersing medium: appropriate amount of water)

| | |
|---|---|
| Particulate titanium dioxide powder | 3 $g/m^2$ |
| Gelatin | 1 $g/m^2$ |
| Octylphenoxypolyethoxyethanol | 0.1 $g/m^2$ |

The surface of the resulting light-reflecting layer was coated with a coating solution for formation of a reagent layer consisting of the following components, and dried to form a first reagent layer.

Components of Coating Solution for First Reagent Layer
(solvent: appropriate amount of water)

| | |
|---|---|
| Cholesterol oxidase | 2,000 U/$m^2$ |
| Gelatin | 10 g/$m^2$ |
| Octylphenoxypolyethoxyethanol | 0.1 g/$m^2$ |
| pH 6.5 Phosphate buffer solution | |

The first reagent layer was wetted with water. A cellulose acetate membrane filter (average pore size 3.0 µm, thickness 180 µm, Microfilter FM—300 (trademark) manufactured by Fuji Photo Film Co., Ltd.) was laminated thereon by slightly pressing it to integrate them, whereby providing a porous spreading layer. Thus, a multilayer chemical-analytical element for quantitative determination of cholesterol.

For evaluating the above multilayer chemical-analytical element, 10 µl. of each of cholesterol-containing control serums (cholesterol content; 50, 100, 200, 300 and 500 mg/dl) and a blank solution containing no cholesterol was spotted on the spreading layer and incubated at 37°C for 10 minutes. Immediately thereafter, the optical density of the formed color was photometrically measured at a measuring wavelength of 520 nm from the PET film side through reflection photometry by means of Macbeth reflection densitometer equipped with a cyan filter. The following results were obtained.

| Cholesterol-Containing Control Serum (content. mg/dl) | Reflection Optical Density |
|---|---|
| 0 (blank) | 0.27 |
| 50 | 0.43 |
| 100 | 0.61 |
| 200 | 0.82 |
| 300 | 0.99 |
| 500 | 1.19 |

It is apparent from the results that the content of cholesterol can be determined by reflection photometry and colorimetry using the above multilayer chemical-analytical element.

**Claims**

1. A reagent for analysis of hydrogen peroxide, which comprises an imidazole derivative having the formula (I):

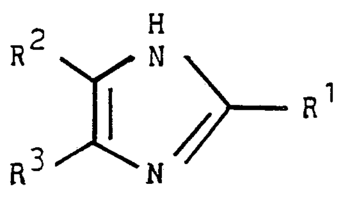

(I)

wherein $R^1$ is an aryl group substituted with hydroxyl group at the ortho- or para-position, $R^2$ is an unsubstituted or substituted aryl group, and $R^3$ is an unsubstituted or substituted alkyl or an unsubstituted alkenyl group.

2. The reagent as claimed in claim 1, which further comprises peroxidase.

3. A method of analysis of hydrogen peroxide which comprises:
reacting hydrogen peroxide with an imdiazole derivative having the formula (I):

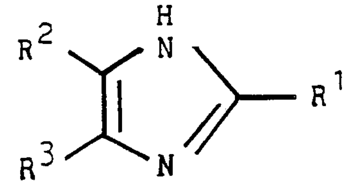

(I)

wherein $R^1$ is an aryl group substituted with hydroxyl group at the ortho- or para-position, $R^2$ is an unsubstituted or substituted aryl group, and $R^3$ is an unsubstituted or substituted alkyl or an unsubstituted alkenyl group, to form a color;
and detecting the formed color.

4. The method as claimed in claim 3, wherein the reaction is carried out in the presence of peroxidase.

5. The method as claimed in claim 3 or 4, wherein the detection of the color is conducted by colorimetry, and the analytical method is a quantitative analysis method.

6. A method of analysis of peroxidase which comprises reacting an imidazole derivative having the formula (I) and hydrogen peroxide in the presence of peroxidase:

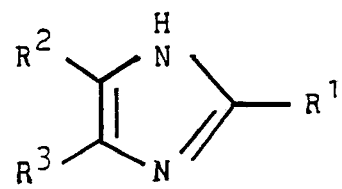

(I)

wherein $R^1$ is an aryl group substituted with hydroxyl group at the ortho- or para-position, $R^2$ is an unsubstituted or substituted aryl group, and $R^3$ is an unsubstituted or substituted alkyl or an unsubstituted alkenyl group.

7. A multilayer chemical-analytical element comprising a water-impermeable, light-transmissive support, a reagent layer and a porous layer superposed in this order,
which is characterized in that an imidazole derivative having the formula (I) is contained in said reagent layer:

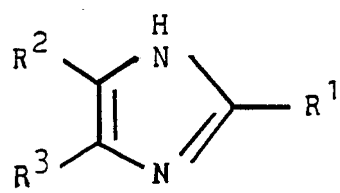

(I)

wherein $R^1$ is an aryl group substituted with hydroxyl group at the ortho- or para-position, $R^2$ is an unsubstituted or substituted aryl group, and $R^3$ is an unsubstituted or substituted alkyl or an unsubstituted alkenyl group.

8. The element as claimed in claim 7 which further contains peroxidase.

9. The element as claimed in claim 7 or 8, wherein said porous layer is a porous spreading layer.

10. The element as claimed in claim 7 or 8, wherein the porous layer is a porous layer having a definite surface area.

**Patentansprüche**

1. Reagens zur Analyse von Wasserstoffperoxid, dadurch gekennzeichnet, daß es ein Imidazolderivat der Formel (I)

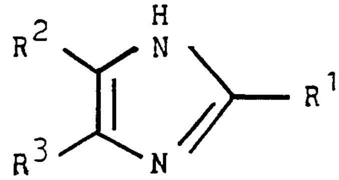

(I)

enthält, worin $R^1$ eine Arylgruppe, die durch eine Hydroxylgruppe an der Ortho- oder Paraposition substituiert ist, bedeutet, $R^2$ eine unsubstituierte oder substituierte Arylgruppe bedeutet und $R^3$ eine unsubstituierte oder substituierte Alkyl- oder eine unsubstituierte Alkenylgruppe bedeutet.

2. Reagens nach Anspruch 1, dadurch gekennzeichnet, daß es weiter Peroxidase enthält.

3. Verfahren zur Analyse von Wasserstoffperoxid, dadurch gekennzeichnet, daß Wasserstoffperoxid mit einem Imidazolderivat der Formel (I) umgesetzt wird,

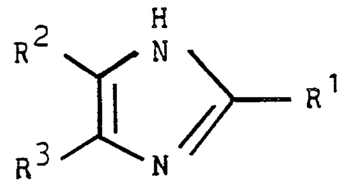

(I)

worin $R^1$ eine Arylgruppe, die durch eine Hydroxylgruppe an der Ortho- oder Paraposition substituiert ist, bedeutet, $R^2$ eine unsubstituierte oder substituierte Arylgruppe bedeutet und $R^3$ eine unsubstituierte oder substituierte Alkyl- oder eine unsubstituierte Alkenylgruppe bedeutet, um einen Farbstoff zu bilden und der gebildete Farbstoff nachgewiesen wird.

4. Verfahren nach Anspruch 3, dadurch gekennzeichnet, daß die Reaktion in Gegenwart von Peroxidase durchgeführt wird.

5. Verfahren nach Anspruch 3 oder 4, dadurch gekennzeichnet, daß der Farbstoff kolorimetrisch detektiert wird und das analytische Verfahren ein quantitativ analytisches Verfahren ist.

6. Verfahren zur Analyse von Peroxidase, durch gekennzeichnet, daß ein Imidazolderivat der Formel (I) und Wasserstoffperoxid in Gegenwart von Peroxidase umgesetzt werden,

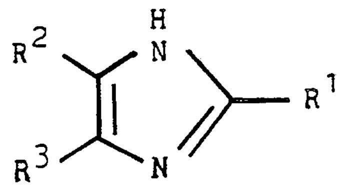

(I)

worin $R^1$ eine Arylgruppe, die durch eine Hydroxylgruppe an der Ortho- oder Paraposition substituiert ist, bedeutet, $R^2$ eine unsubstituierte oder substituierte Arylgruppe bedeutet und $R^3$ eine unsubstituierte oder substituierte Alkyl- oder eine unsubstituierte Alkenylgruppe bedeutet.

7. Mehrschichtiges chemisches Analysenelement aus einem wasserundurchlässigen, lichtdurchlässigen Träger, einer Reagensschicht und einer porösen Schicht, die in dieser Reihenfolge übereinandergelegt sind, dadurch gekennzeichnet, daß ein Imidazolderivat der Formel (I) in der genannten Reagensschicht enthalten ist,

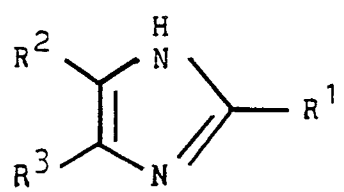

(I)

worin $R^1$ eine Arylgruppe, die durch eine Hydroxylgruppe an der Ortho- oder Paraposition substituiert ist, bedeutet, $R^2$ eine unsubstituierte oder substituierte Arylgruppe und $R^3$ eine unsubstituierte oder substituierte Alkyl- oder eine unsubstituierte Alkenylgruppe bedeuten.

8. Element nach Anspruch 7, dadurch gekennzeichnet, daß es weiter Peroxidase enthält.

9. Element nach Anspruch 7 oder 8, dadurch gekennzeichnet, daß die genannte pörose Schicht eine poröse Ausbreitungsschicht ist.

10. Element nach Anspruch 7 oder 8, dadurch gekennzeichnet, daß die poröse Schicht eine poröse Schicht mit einer definierten Oberfläche ist.

**Revendications**

1. Réactif pour l'analyse du peroxyde d'hydrogène, caractérisé en ce qu'il comprend un dérivé d'imidazole ayant la formule (I):

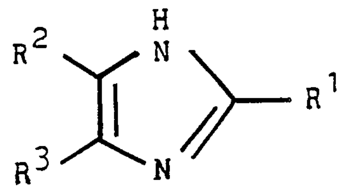

(I)

dans laquelle $R^1$ est un groupe aryle substitué avec un groupe hydroxy en position ortho ou para, $R^2$ est un groupe aryle non substitué ou substitué et $R^3$ est un groupe alkyle non substitué ou substitué ou un groupe alcényle non substitué.

2. Réactif suivant la revendication 1, caractérisé en ce qu'il comprend de plus une peroxydase.

3. Procédé d'analyse du peroxyde d'hydrogène, caractérisé en ce qu'il comprend:

la réaction du peroxyde d'hydrogène avec un dérivé d'imidazole ayant la formule (I):

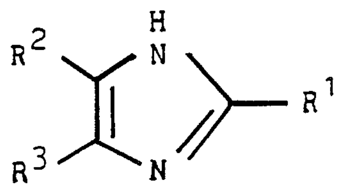

(I)

dans laquelle $R^1$ est un groupe aryle substitué avec un groupe hydroxy en position ortho ou para, $R^2$ est un groupe aryle non substitué ou substitué et $R^3$ est un groupe alkyle non substitué ou substitué ou un groupe alcényle non substitué, de façon à former une couleur;

et la détection de la couleur formée.

4. Procédé suivant la revendication 3, caractérisé en ce que la réaction est effectuée en présence d'une peroxydase.

5. Procédé suivant la revendication 3 ou la revendication 4, caractérisé en ce que la détection de la couleur est effectuée par colorimétrie et que le procédé analytique est un procédé d'analyse quantitative.

6. Procédé d'analyse de peroxydase, caractérisé en ce qu'il comprend la réaction d'un dérivé d'imidazole ayant la formule (I) et de peroxyde d'hydrogène en présence de peroxydase:

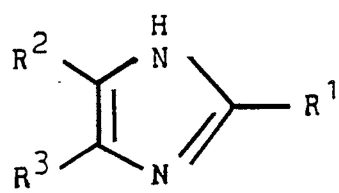

(I)

dans laquelle $R^1$ est un groupe aryle substitué avec un groupe hydroxy en position ortho ou para, $R^2$ est un groupe aryle non substitué ou substitué et $R^3$ est un groupe alkyle non substitué ou substitué ou un groupe alcényle non substitué.

7. Elément chimico-analytique à plusieurs couches comprenant un support imperméable à l'eau et qui transmet la lumière, une couche de réactif et une couche poreuse superposés dans cet ordre, caractérisé en ce qu'un dérivé d'imidazole ayant la formule (I) est contenu dans cette couche de réactif:

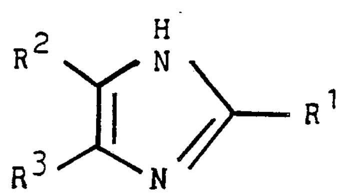

(I)

dans laquelle $R^1$ est un groupe aryle substitué avec un groupe hydroxy en position ortho ou para, $R^2$ est un groupe aryle non substitué ou substitué et $R^3$ est un groupe alkyle non substitué ou substitué ou un groupe alcényle non substitué.

8. Elément suivant la revendication 7, caractérisé en qu'il contient de plus une peroxydase.

9. Elément suivant la revendication 7 ou la revendication 8, caractérisé en que cette couche poreuse est une couche d'étalement poreuse.

10. Elément suivant la revendication 7 ou la revendication 8, caractérisé en que cette couche poreuse est une couche poreuse ayant une aire superficielle définie.